Europäisches Patentamt

European Patent Office

Office européen des brevets

(19)

(11) Numéro de publication : **0 171 319**
**B1**

(12) # FASCICULE DE BREVET EUROPÉEN

(45) Date de publication du fascicule du brevet :
25.05.88

(21) Numéro de dépôt : 85401410.7

(22) Date de dépôt : 11.07.85

(51) Int. Cl.⁴ : **C 12 M   1/34, G 01 N 35/00,**
**G 01 N  21/25, C 12 M   1/26**

(54) Automate pour l'analyse et le clonage de cultures cellulaires ainsi que pour l'analyse bactériologique.

(30) Priorité : 12.07.84 FR 8411073

(43) Date de publication de la demande :
12.02.86 Bulletin 86/07

(45) Mention de la délivrance du brevet :
25.05.88 Bulletin 88/21

(84) Etats contractants désignés :
AT BE CH DE FR GB IT LI LU NL SE

(56) Documents cités :
FR-A- 2 250 991
FR-A- 2 424 961
GB-A- 1 205 564
US-A- 3 322 956
US-A- 3 620 678
US-A- 3 773 426

(73) Titulaire : **Bisconte, Jean-Claude**
**35 rue du Professeur Pauchet**
**F-92420 Vaucresson (FR)**

(72) Inventeur : **Bisconte, Jean-Claude**
**35, rue du Professeur Pauchet**
**F-92420 Vaucresson (FR)**

(74) Mandataire : **Orès, Bernard et al**
**Cabinet ORES 6, Avenue de Messine**
**F-75008 Paris (FR)**

Jouve, 18, rue St-Denis, 75001 Paris, France

**Description**

Cette invention est relative à un automate pour l'analyse et le clonage de cultures cellulaires, ainsi que pour l'analyse bactériologique, à savoir à un appareil intégrant les phases d'entretien, d'expérimentation de substances, notamment pharmacologiques, d'analyse optique de la densité et de la forme des cellules, ainsi que de leurs associations, et de repiquage des cellules adhérentes en vue de leur clonage.

Actuellement, la culture cellulaire sur support est pratiquée dans des récipients standardisés tels que boîtes de Petri, boîtes multitrous, ou flacons. L'analyse morphologique des cellules vivantes, pratiquée généralement sur microscope inversé en contraste de phase, a pour but de dénombrer les cellules, de repérer la présence de colonies, d'étudier la différenciation spontanée ou provoquée des cellules, ou encore de suivre l'évolution de leur forme et de leur mouvement par micro-cinéma (time-lapse cinematography).

Ces observations sont d'un très grand intérêt pour étudier la croissance et la différenciation normale ou expérimentale de cellules, en particulier pour suivre les effets de drogues, hormones, produits toxiques. L'utilisation des récipients actuels implique l'exécution de phases constituant une séquence d'opérations dissociées, avec le passage dans des dispositifs variés, tels que : étuves humides à $CO_2$ pour le stockage, hottes à flux laminaires pour le renouvellement des milieux ou les manipulations de cellules, microscope inversé couplé ou non à un dispositif d'imagerie (caméra vidéo ou caméra photographique).

Plusieurs constructeurs de microscope proposent des enceintes thermostatées comprenant un microscope inversé.

Il existe, par ailleurs, des platines motorisées et des dispositifs d'analyse d'image.

L'inventeur a, pour sa part, montré en 1980 (MIKROSCOPIE) l'utilisation de l'analyse d'image vidéo, associée à une enceinte thermostatée et à des platines motorisées, pour suivre la cinétique cellulaire.

De tels dispositifs de grand volume, associant des composants classiques, peuvent comporter une régulation de la teneur en $CO_2$, mais il n'est pas opportun de créer une saturation en eau qui provoquerait inévitablement des zones de condensation et serait source de contamination, mais surtout de détérioration rapide des optiques et de la micromécanique. Ainsi, l'évaporation rapide qui en résulte limite la durée des séquences d'analyse à quelques heures et 48 heures au maximum.

De plus, les formes complexes des dispositifs inclus dans l'enceinte ne sont pas appropriées au maintien de la stérilité.

Actuellement, ces enceintes, qui incluent un microscope, permettent de suivre l'évolution de cultures maintenues dans les puits d'une boîte, mais le passage à l'analyse en temps limité de plusieurs boîtes, et a fortiori l'analyse à long terme, c'est-à-dire incluant des opérations de changement du milieu nutritif, ne sont pas possibles d'une façon automatisée.

L'inventeur a proposé par ailleurs une solution originale autorisant l'entretien et l'analyse à long terme de boîtes de cultures de formes classiques (Cf. la Demande de Brevet en France n° 84 08839).

On connaît également le Brevet US-3 773 426 qui décrit un détecteur de croissance bactérienne, qui utilise une pluralité de faisceaux lumineux et de capteurs capables d'effectuer simultanément des tests sur une pluralité d'échantillons et de déterminer les résultats électroniquement tout en les emmagasinant sur des cartes mémoires.

De façon plus précise, le détecteur susdit comporte en combinaison :

des moyens de support permettant la croissance des bactéries et constitués par une pluralité de puits destinés à recevoir une solution de croissance bactérienne dont la concentration est différente dans chaque puits, de façon à permettre le titrage en série de la croissance bactérienne ;

des capteurs d'identification des solutions contenues dans les puits et de mesure de leur densité lorsque le moyen de support précité fait passer les puits de titrage devant ces capteurs, et

des moyens de traitement de données connectés électriquement aux capteurs pour effectuer automatiquement les calculs de densité et indiquer le point final du titrage en série.

On connaît encore la Demande de Brevet FR-2 424 961 qui décrit un dispositif pour la mise en œuvre d'un procédé d'inoculation multiple simultanée d'échantillons bactériologiques.

Ce dispositif permet essentiellement le transfert simultané de bactéries d'un grand nombre de cellules d'un bac à essais à un grand nombre de cellules d'un autre bac dit bac à préparation, et ce en un seul processus sans qu'aucune stérilisation intermédiaire ne soit nécessaire.

Lorsque les opérations d'entretien, de prélèvement et d'analyse cellulaire ne sont pas à très long terme, ne mettent pas en œuvre un très grand nombre d'échantillons et ne présentent pas de grands risques de contamination pour l'homme, il est intéressant de concevoir un appareil strictement adapté et optimisé dans tous ses composants.

Le but premier de l'invention est d'éliminer ces inconvénients, en particulier en ce qui concerne la durée limitée d'analyse, le nombre restreint de logettes qu'on peut traiter et des champs d'observation qu'on peut examiner, ainsi que les risques de contamination.

Un autre but de l'invention est de fournir un appareil original qui intègre des opérations qui jusqu'à présent sont dissociées, notamment en ce qui concerne le clonage automatique.

Un autre but de l'invention est de fournir un appareil unique et autonome relativement peu coûteux qui n'exige pas l'investissement en salles et en équipements spécialisés.

Un autre but de l'invention est de se rapprocher des conditions optimales en supprimant totalement les chocs thermiques et mécaniques, et en réalisant des conditions de culture uniformes indépendamment de l'opérateur.

Un autre but de l'invention est de permettre d'asservir, par des logiciels, les conditions d'environnement des cellules, y compris le milieu nutritif, à des paramètres mesurés fréquemment ou même en continu.

La présente invention a pour objet un automate très compact pour l'analyse et le clonage de cultures cellulaires, ainsi que pour l'analyse bactériologique, à savoir un appareil intégrant les phases d'entretien, d'expérimentation de substances notamment pharmacologiques, d'analyse optique de la densité et de la forme, ainsi que de leurs associations, et de repiquage de cellules en vue de leur clonage, caractérisé en ce qu'il comprend, en combinaison :

- une enceinte à parois isolantes, assurant l'homéothermie et certaines conditions d'ambiance, notamment en termes d'hygrométrie, pourcentage de $CO_2$ et/ou autre, à l'intérieur de cette enceinte, qui est divisée en deux compartiments, dont le premier compartiment est à faible volume et contient :

un plateau, de préférence circulaire, comprenant plusieurs micro-puits disposés suivant une ou plusieurs rangées circulaires concentriques, le plateau et les micro-puits étant réalisés en une matière transparente ayant de bonnes qualités optiques,

au moins un dispositif spécial d'observation microscopique, notamment du type à contraste de phase,

un dispositif d'aspiration/injection de milieu cellulaire ou de cellules, garantissant la non contamination,

un bloc moteur assurant l'entraînement en rotation ainsi que les déplacements vertical et horizontal du plateau, avec positionnement très précis dans les trois directions de mouvement, tandis que le deuxième compartiment de l'enceinte, qui enveloppe le premier compartiment sauf au niveau de la face avant de l'enceinte, contient un dispositif de régulation de la température et de brassage de l'air chaud et, éventuellement, un dispositif de stérilisation du premier compartiment par flux d'air laminaire, filtré et en circuit fermé, connu en soi, lesdits premier et deuxième compartiments étant séparés par une paroi, éventuellement filtrante, pour permettre la circulation entre ces deux compartiments de l'éventuel flux laminaire, et

- un bloc réfrigérateur disposé à l'extérieur de l'enceinte ainsi équipée, contenant en particulier les réservoirs d'alimentation en milieux nutritifs et différents produits.

Selon un mode de réalisation avantageux de l'automate conforme à l'invention, ledit plateau est pourvu d'une ouverture centrale d'accès dudit dispositif d'aspiration/injection, qui est fixé à la paroi supérieure du premier compartiment, dans un puits d'évacuation et de stérilisation fixé à la paroi inférieure de ce compartiment.

Selon un autre mode de réalisation avantageux de l'automate conforme à l'invention, ledit plateau circulaire est placé dans une cassette, notamment métallique et de forme sensiblement carrée qui, lorsqu'elle est équipée d'un couvercle, réalise une configuration proche de la boîte de Petri propre à réduire les risques de contamination et qui comporte un évidement circulaire central de logement du plateau qui, en position de repos, est tenu en place par des moyens de support constitués, de préférence, par quatre billes logées dans la paroi dudit évidement et dépassant de cette dernière pour s'encliqueter dans une gorge annulaire ménagée dans la paroi extérieure du plateau, tandis que l'entraînement en rotation du plateau est réalisé à l'aide d'un galet moteur, faisant saillie dudit bloc moteur, par engagement sous l'action de moyens appropriés et par frottement dans la gorge du plateau, à cet effet une fenêtre d'accès du galet à cette gorge étant ménagée à la partie médiane, du côté de la cassette adjacent au bloc moteur.

Selon une disposition avantageuse de ce mode de réalisation, deux desdites billes sont fixes tandis que les deux autres billes sont sur support élastique, notamment à ressort, les quatre billes étant disposées aux sommets d'un carré inscrit dans la circonférence délimitant ledit évidement circulaire, de manière qu'un côté de ce carré, parallèle et contigu à ladite fenêtre d'accès du galet moteur, est défini par les deux billes fixes, tandis que le côté opposé du carré est défini par lesdites deux billes sur support élastique, en sorte que, lors de l'engagement du galet dans ladite gorge, ces deux dernière billes seulement participent au guidage en rotation du plateau, les deux billes fixes venant se dégager de la gorge de ce dernier.

Selon une autre disposition avantageuse de ce mode de réalisation, lesdits moyens permettant l'engagement du galet moteur dans la gorge du plateau sont constitués par un électro-aimant réalisant l'accouplement souple entre les parois métalliques opposées du bloc moteur et de la cassette.

Selon encore une autre disposition avantageuse de ce mode de réalisation, la cassette de support est pourvue d'un couvercle transparent, de bonne qualité optique, s'adaptant à ladite ouverture centrale du plateau et pourvue d'une fenêtre radiale d'accès dudit dispositif d'aspiration/injection aux micro-puits du plateau.

Egalement conformément à l'invention, des billes disposées sur les parois latérales et sur les parois inférieure et supérieure de la cassette permettent le libre glissement de cette cassette de support.

Encore également conformément à l'invention, la cassette de support est stérilisable, de même que le plateau et le couvercle.

Selon un mode de réalisation préféré de l'automate conforme à l'invention, celui-ci coopère avec une pluralité de plateaux, chacun contenu dans une cassette du type susdit, de manière à stocker les cassettes dans une étuve à part et à n'introduire dans ledit premier compartiment que la cassette correspondant à un certain protocole d'expérience.

Selon encore un autre mode de réalisation avantageux de l'automate conforme à l'invention, ledit bloc moteur comprend en combinaison :

- un premier moteur pas-à-pas d'entraînement en rotation dudit plateau, qui agit sur ledit galet moteur par l'intermédiaire d'un système réducteur,

- un deuxième moteur pas-à-pas qui assure le déplacement horizontal du plateau en agissant sur un système d'engrenage qui fait saillie du bloc moteur et qui agit à son tour sur au moins une crémaillère fixée à la paroi inférieure du premier compartiment, et

- un troisième moteur pas-à-pas de réglage unilatéral de la hauteur du plateau, qui agit sur une vis micrométrique disposée latéralement par rapport audit galet moteur et pourvue à son extrémité inférieure d'une bille de roulement glissant sur la paroi inférieure dudit premier compartiment, de manière à permettre les déplacements horizontaux commandés par ledit deuxième moteur pas-à-pas.

Selon un mode de réalisation avantageux de l'automate conforme à l'invention, ledit dispositif d'observation microscopique comporte deux couples objectif/condenseur préréglés, qui sont associés entre eux et qui sont destinés à fonctionner alternativement, les premier et deuxième condenseurs et les premier et deuxième objectifs étant solidaires du plafond et du plancher, respectivement, de la paroi de séparation entre lesdits deux compartiments de l'enceinte, lequel premier objectif est à faible grossissement, tandis que le deuxième objectif assure un grossissement moyen ou fort en fonction des besoins, l'arrivée de lumière dans les deux condenseurs se faisant par un système à fibres optiques.

Selon une disposition avantageuse de ce mode de réalisation, chaque objectif coopère avec un miroir et un capteur d'images constitué par une caméra TV ou par une barrette CCD couplé ou non à un analyseur d'images automatique.

Selon encore une autre modalité avantageuse de cette disposition, le miroir intermédiaire, qui est interposé entre ledit capteur d'images et le miroir le plus éloigné de ce dernier, est semi-transparent.

Selon une variante avantageuse de cette modalité, ledit miroir intermédiaire est escamotable, notamment sous l'action d'un système à électro-aimant, lorsque le couple objectif/condenseur le plus éloigné du capteur d'images est activé.

Selon un autre mode de réalisation avantageux de l'automate conforme à l'invention, le dispositif d'aspiration/injection est disposé dans un logement vertical ménagé dans la paroi supérieure du premier compartiment, et coopère avec un dispositif de réglage à trois positions de la hauteur de l'embout d'aspiration/injection, notamment constitué par un moteur pas-à-pas ou par un vérin télescopique, permettant :

- en position haute, de faire déplacer librement la cassette de support ainsi que le plateau,

- en position intermédiaire, d'aspirer du milieu cellulaire appauvri, ou des cellules, ou d'injecter du milieu enrichi ou des cellules dans un puits du plateau, l'éventuel contact entre la pointe dudit embout et le fond du puits étant obtenu par l'action dudit troisième moteur pas-à-pas de réglage de la hauteur du plateau, et

- en position basse, d'évacuer dans ledit puits d'évacuation, fixé à la paroi inférieure du premier compartiment, le milieu appauvri aspiré et de procéder aux rinçages et à la stérilisation.

Selon une disposition avantageuse de ce mode de réalisation, l'embout d'aspiration/injection est pourvu d'une collerette faisant saillie de sa paroi externe qui, dans ladite position basse du dispositif d'aspiration/injection, s'applique par pression sur le bord supérieur dudit puits d'évacuation pour assurer l'étanchéité vis-à-vis des opérations de rinçage et de stérilisation de la partie externe de l'embout.

Selon une variante avantageuse de ce mode de réalisation, lorsque le dispositif de réglage de la hauteur de l'embout d'aspiration/injection est en position intermédiaire, ce dernier s'applique, au moyen de ladite collerette, sur le puits d'évacuation qui est contenu dans le même logement du dispositif d'aspiration/injection, le puits d'évacuation étant porté par pivotement ou par glissement au-dessous de l'embout ainsi positionné par tous moyens appropriés, tandis que les opérations d'aspiration/injection s'effectuent en position basse.

Selon encore un autre mode de réalisation avantageux de l'automate conforme à l'invention, un dispositif d'injection séparé est supporté par la collerette d'étanchéité, solidaire de l'embout dudit dispositif d'aspiration, et comprend une arrivée d'air stérile et chaud qui crée une colonne d'air laminaire autour de l'arrivée de milieu nutritif injecté par des clapets et qui empêche le reflux de particules contaminantes, à savoir les risques de contamination rétrograde.

Selon une disposition avantageuse de ce mode de réalisation, le dispositif d'injection coopère avec un dispositif de contrôle de niveau du milieu liquide injecté dans chaque micro-puits, comportant une fibre optique portée par ladite collerette et, en-dessous de chaque micro-puits, une diode.

Selon un autre mode de réalisation avantageux de l'automate conforme à l'invention, il coopère avec un microordinateur de pilotage de tous les dispositifs et de toutes les opérations qu'ils doivent accomplir suivant le protocole d'expérience prédéterminé, et qui assure également le traitement éventuel des données.

Outre les dispositions qui précèdent, l'invention comprend encore d'autres dispositions, qui ressortiront de la description qui va suivre.

L'invention sera mieux comprise à l'aide du complément de description qui va suivre, qui se réfère aux dessins annexés dans lesquels :

- la figure 1 montre une vue schématique en coupe de l'appareil conforme à l'invention ;
- la figure 2 est une vue en perspective de la cassette selon l'invention destinée à supporter le plateau conforme à l'invention ;
- la figure 3 montre le détail de la liaison entre la cassette représentée à la figure 2 et le plateau selon l'invention ;
- la figure 4 montre en perspective la cassette avec le plateau et son couvercle et le bloc moteur conformes à l'invention, disposés dans le compartiment qui les contient ainsi que l'emplacement des dispositifs d'aspiration/injection et d'observation microscopique également conformes à l'invention ;
- la figure 5a montre également en perspective le bloc moteur selon l'invention ;
- la figure 5b montre une vue en coupe et en élévation du bloc moteur de la figure 5a, avec le galet moteur d'entraînement en rotation du plateau, selon l'invention, engagé dans la gorge annulaire de ce dernier ;
- la figure 6 représente le dispositif d'observation microscopique selon l'invention ;
- la figure 7 montre le dispositif d'injection couplé au dispositif d'aspiration selon l'invention, et
- les figures 8 et 9 illustrent une variante du plateau conforme à l'invention.

Ces dessins et les parties descriptives correspondantes illustrent l'invention.

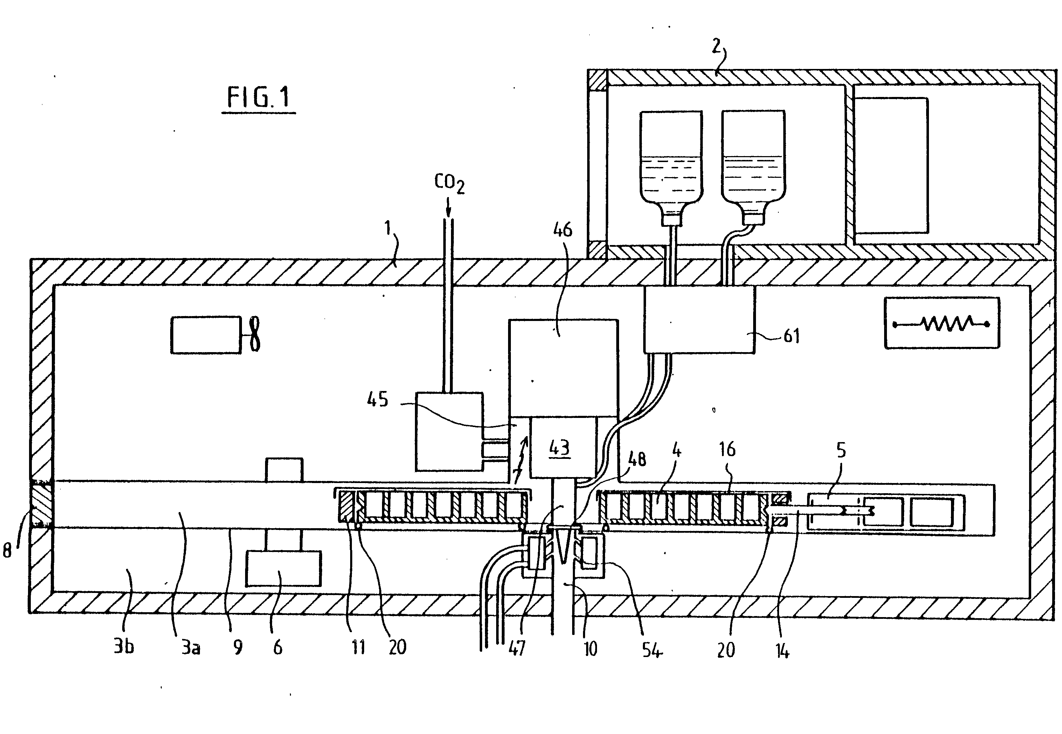

L'automate selon l'invention comporte deux blocs principaux (Cf. la figure 1) :

- l'enceinte 1 proprement dite, et
- un bloc refroidi 2 qui contient en particulier les milieux nutritifs.

L'enceinte 1, à parois isolantes, est divisée en deux compartiments :

- le compartiment 3a, qui contient un plateau 4, un bloc moteur 5, le dispositif d'observation microscopique 6 et un dispositif d'aspiration/injection 7, et
- le compartiment 3b, qui enveloppe complètement le précédent, sauf au niveau de la porte d'accès 8 ménagée dans la face avant de l'enceinte 1, ces compartiments étant séparés par une paroi métallique 9 en acier ou en aluminium anodisé, par exemple.

Dans la configuration représentée à la figure 1, une circulation d'air forcée est établie entre les deux compartiments 3a et 3b, et un filtre (non représenté) est interposé afin de retenir les particules et les germes de contamination.

Le flux d'air stérile et chaud, ainsi obtenu, traverse des orifices (non représentés) qui peuvent être ménagés sur le fond du compartiment 3a. Ce flux peut être repris par des orifices (non représentés) qui communiquent avec ledit filtre.

Le plateau 4 est réalisé en matériau transparent de bonne qualité optique, tel que le polyméthacrylate ou le verre. D'un diamètre extérieur de 25 centimètres, le plateau 4 peut contenir, sur sa rangée externe, par exemple, 16 micro-puits ayant chacun un diamètre de 35 mm ou encore 48 micro-puits de 12 mm de diamètre.

Le plateau 4 est avantageusement pourvu d'une ouverture centrale d'accès dudit dispositif d'aspiration/injection dans un puits d'évacuation 10 fixé à la paroi inférieure du premier compartiment 3a.

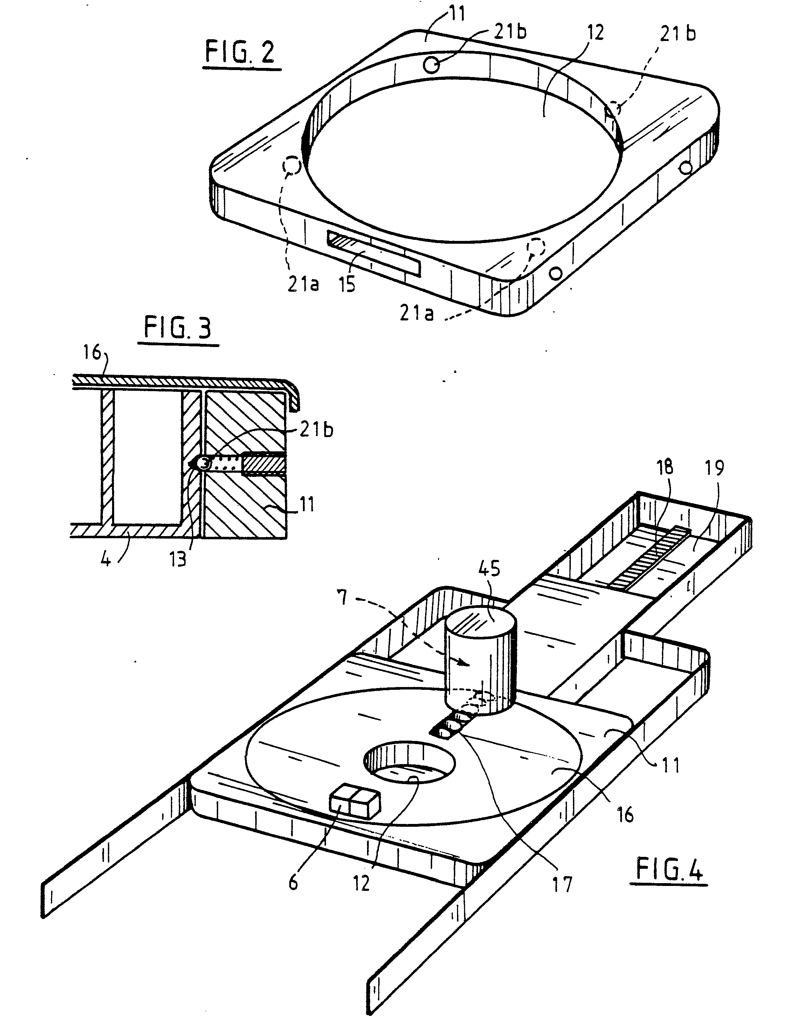

Le plateau est disposé à l'intérieur d'une cassette stérilisable 11, notamment métallique (Cf. la figure 2), de forme carrée et à angles arrondis, qui comporte un évidement circulaire central 12 de logement du plateau 4.

En position de repos, ce plateau est maintenu par des moyens de support constitués de préférence par quatre billes 21a et 21b, logées dans la paroi dudit évidement 12 et dépassant de cette dernière pour s'encliqueter dans une gorge annulaire 13 (Cf. la figure 3) ménagée dans la paroi latérale du plateau 4. Ces quatre billes sont disposées aux sommets d'un carré inscrit dans la circonférence délimitant ledit évidement circulaire 12 : les deux billes 21a définissant le côté du carré qui se trouve à proximité du bloc moteur 5 sont fixes, tandis que les deux billes 21b définissant le côté opposé de ce carré sont reliées à un support élastique à ressort.

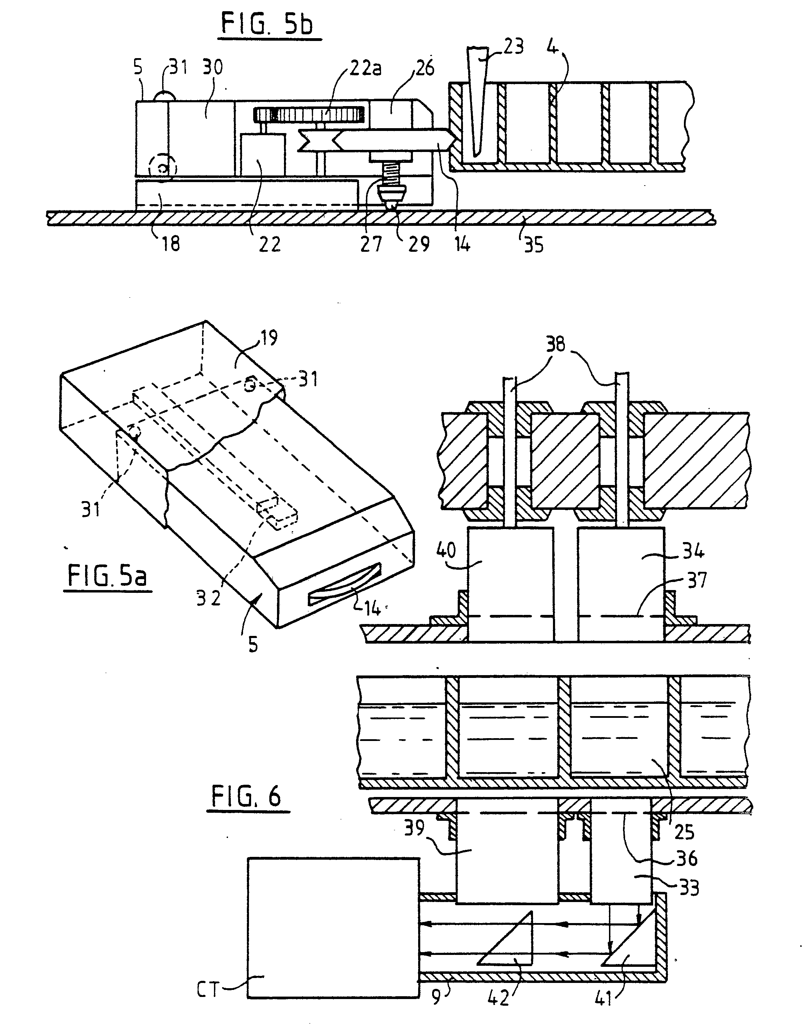

L'entraînement en rotation du plateau 4 est obtenu par frottement d'un galet moteur 14 (Cf. la figure 5b) dans la gorge 13 à profil triangulaire ou trapézoïdale du plateau 4, dans laquelle il est engagé sous l'action d'un électro-aimant (non représenté) qui réalise l'accouplement souple entre les parois métalliques opposées du bloc moteur 5 et de la cassette 11 : à cet effet, une fenêtre 15 d'accès du galet 14 dans la gorge 13 est ménagée à la partie médiane du côté de la cassette 11 adjacent au bloc moteur 5. Lors de cet accouplement, l'engagement du galet moteur 14 dans la gorge 13 du plateau 4, pousse ce dernier contre les deux billes de support 21b, qui sont disposées à l'opposé de la fenêtre 15 et de part et d'autre par rapport à l'axe transversal de cette dernière et participent, ainsi, également au guidage en rotation du plateau, tandis que les deux billes de support fixes 21a se dégagent de sa gorge 13.

La cassette 11 est pourvue d'un couvercle 16 transparent et de bonne qualité optique, s'adaptant à ladite ouverture centrale du plateau 4 permettant l'accès au dispositif d'aspiration/injection dans ledit puits d'évacuation 10.

Ce couvercle 16 est pourvu d'une fenêtre radiale 17 d'accès de ce dispositif d'aspiration/injection aux micro-puits ménagés dans le plateau 4 et alignés radialement.

Il va de soi qu'on peut prévoir une pluralité de cassettes 11, chacune comportant son propre plateau 4, de manière à faciliter le stockage, notamment dans une étuve séparée, et à n'introduire dans le premier compartiment 3a, par la porte isotherme 8 de fermeture de la face avant de l'enceinte 1, que la cassette correspondant au protocole d'expérience prédéterminé ou la cassette désirée.

De toutes façons, il faut également tenir compte de l'avantage qui découle de la possibilité de réutiliser chaque plateau après un premier emploi.

Le glissement de la cassette 11 à l'intérieur du compartiment 3a se fait à l'aide de billes 20 disposées sur la paroi inférieure de la cassette, ainsi que sur ses parois latérales et supérieure. La figure 4 montre, en perspective, la configuration du compartiment 3a, qui est conçu de façon à rendre possibles en particulier les déplacements horizontaux de la cassette 11, avec son plateau 4, à l'aide du bloc moteur 5, comme il est décrit plus loin : cette figure 4 montre les parois latérales et inférieure du compartiment 3a, la paroi supérieure étant enlevée pour rendre le dessin plus clair et montrer les composants qui y sont contenus à l'intérieur ; dans cette figure 4 on peut voir aussi un élément dénoté par la référence 45 (cf. aussi la figure 1), qui constitue en particulier un logement pour le dispositif d'aspiration/injection (qui, dans la figure 4, est caché par le logement 45 et est dénoté par le chiffre 7 associé à une ligne de référence en pointillé), ainsi que le dispositif optique 6, qui sont solidaires de la paroi supérieure du compartiment 3a.

Le galet 14 est actionné par un moteur pas-à-pas 22 (Cf. la figure 5b) par l'intermédiaire d'un système réducteur 22a. La précision de positionnement en rotation du plateau 4 est de l'ordre de 10 µm, soit de l'ordre de la taille d'une cellule. La fréquence du moteur 22 est, par exemple, de l'ordre de 500 Hz et la vitesse de rotation du plateau 4 est d'environ 5 mm.s$^{-1}$, soit une rotation complète en deux minutes et trente secondes. La rotation est possible dans les deux sens.

Il est parfois nécessaire de faire varier la hauteur du plateau 4, par exemple pour mettre en contact l'aiguille 23 du dispositif d'aspiration 7 avec les cellules 24 adhérant au fond d'un micro-puits 25 ou, également, pour parfaire la mise au point optique sachant que le dispositif microscopique (Cf. la figure 6) est fixe et préréglé. Dans ces cas, l'amplitude des mouvements verticaux est au maximum de 2 millimètres : à cet effet, on peut utiliser un moteur 26 pas-à-pas de réglage unilatéral de la hauteur du plateau 4, qui agit sur une vis micrométrique 27 disposée latéralement par rapport audit galet moteur 14 et pourvue à son extrémité inférieure d'une bille de roulement 29 glissant sur le plancher interne 35 de séparation entre lesdits deux compartiments 3a et 3b.

De même, il est prévu encore un autre moteur pas-à-pas 30 pour les déplacements horizontaux.

Ces trois moteurs sont réunis entre eux et constituent ledit bloc moteur 5.

Pour obtenir le déplacement horizontal du plateau 4 pris entre le galet moteur 14 et les billes 21b enfoncées en butée, on fait agir le moteur pas-à-pas 30 sur un engrenage (non représenté) faisant saillie du bloc moteur 5 et agissant à son tour sur une crémaillère 18 fixée à la paroi inférieure du logement 19 dans lequel se déplace le bloc moteur 5 en glissant à frottement doux contre les parois latérales de ce logement 19. Un évidement 32 entaille le bloc moteur 5 dans sa partie intérieure. Des billes 31 assurent le glissement contre la face supérieure du logement 19.

Avantageusement, les trois moteurs 22, 26 et 30 du bloc moteur 5 sont du type étanche de manière à résister à l'humidité qui règne dans le premier compartiment 3a.

Grâce au bloc moteur 5 selon l'invention, différents types de balayage des micro-puits 25 sont possibles, par exemple :
- un balayage selon un tracé continu à faible grossissement, ou
- un balayage selon un tracé continu à fort grossissement, ou encore
- un balayage selon un tracé en zig-zag quelconque. Alors que les balayages en continu permettent d'obtenir des profils densitométriques, les balayages en zig-zag consistent dans une suite d'analyses de champs préprogrammés en aléatoire ou correspondant à des repérages individuels.

On comprend aisément que le principe même de l'automate permet de repérer automatiquement les micro-puits par l'alternance de zones sombres dans les profils densitométriques obtenus selon le tracé choisi pour le balayage des micro-puits : ainsi, un recalage précis peut être fait en cas d'erreur de programme ou de panne momentanée. Par ailleurs, on peut faire le zéro des mesures en obscurcissant le fond d'un micro-puits ou en plaçant des repères optiques.

L'analyse optique des cellules contenues dans les micro-puits 25 et adhérant au fond de ces derniers est réalisée par des couples objectif/condenseur à contraste de phase pré-réglés. Dans un grand nombre d'applications, un seul couple objectif/condenseur est nécessaire. Dans l'exemple choisi, deux couples sont associés et placés tangentiellement par rapport à l'axe circulaire passant par les points milieux des micro-puits de la rangée à analyser, comme le montre la figure 4. L'objectif 33 à faible grossissement (10 x) est placé en alignement d'un condenseur 34 qui contient les anneaux de phase correspondants 36 et 37. L'arrivée de lumière dans les condenseurs se fait par un système à fibres optiques 38. De la même façon, l'objectif 39 de grossissement moyen ou fort (32 x ou 50 x) est en alignement du condenseur 40.

Les deux couples 33-34 et 39-40 fonctionnent en alternance. Lorsque l'observation se fait à faible grossissement avec l'objectif 33, un prisme extérieur (non représenté) envoie un faisceau de lumière sur la fibre optique correspondante. Le plan focal correspond au fond d'un micro-puits 25. La mise au point peut être modifiée, comme expliqué précédemment, par les déplacements verticaux du plateau 4. En sortant de l'objectif 33, l'image est renvoyée par un miroir 41 vers la caméra de télévision CT placée soit dans le compartiment 3b, soit à l'extérieur en rallongeant le guide de lumière g. Au passage, les rayons lumineux traversent le miroir 42 semi-transparent. Pour l'observation à fort grossissement du même champ cellulaire, le galet 14 fait tourner le plateau 4 d'une quantité équivalente à un secteur angulaire correspondant au pas fixé. Avec la disposition tangentielle susdite des couples optiques, on peut aisément aligner un, deux ou trois couples, voire plus, et disposer d'un dispositif d'observation d'une grande polyvalence puisqu'il n'est pas nécessaire de faire tourner la tourelle d'un microscope pour changer de grossissement.

Au lieu d'utiliser un miroir intermédiaire semi-transparent 42, on peut avantageusement disposer un miroir escamotable (non représenté), notamment sous l'action d'un système à électro-aimant, lorsque le couple objectif/condenseur 33-34 est activé.

L'analyse optique peut se faire visuellement sans caméra, ou avec caméra TV, ou avec barrette CCD, ou en automatique par un analyseur d'image couplé à un quelconque de ces capteurs d'images.

Le dispositif d'observation optique 6, ainsi que la caméra CT, sont disposés à l'extérieur du premier compartiment 3a qui peut être rendu humide sans inconvénients, en protégeant les condenseurs et les objectifs par des plaques en verre (non représentées) encastrées dans le plafond et le plancher du compartiment 3a au niveau des ouvertures ménagées dans ces parois en correspondance de l'emplacement desdits condenseurs et objectifs ; à cet effet, il y a lieu de considérer que le faible volume de ce compartiment 3a assure une humidification automatique et constante par évaporation du milieu cellulaire contenu dans les micro-puits 25.

Le dispositif d'aspiration 7 est du type décrit dans ladite Demande de Brevet n° 84 08839, et se compose d'un système à piston comprenant un moteur pas-à-pas et un système réducteur, contenus dans le bloc 43, lequel système réducteur agit sur un piston contenu dans un cylindre 44.

Le dispositif d'aspiration/injection 7 est disposé dans un logement vertical 45 (Cf. les figures 1 et 4), ménagé dans la paroi supérieure du premier compartiment 3a, et coopère avec un dispositif 46 de réglage de la hauteur de l'embout 47, à trois positions. Ce dispositif 46 peut être constitué par un moteur pas-à-pas ou par un vérin du type télescopique et permet d'effectuer les opérations suivantes :

- en position haute, de faire déplacer librement ladite cassette de support 11, ainsi que son plateau 4 ;

- en position intermédiaire, de pourvoir à l'aspiration ou à l'injection, et

- en position basse, d'évacuer dans ledit puits d'évacuation le ou les produits aspirés.

L'opération d'évacuation peut avantageusement être suivie par une opération de rinçage et de stérilisation de la surface externe de l'embout 47 : à cet effet, ce dernier est pourvu d'une collerette 48 qui permet son application par pression, en position basse, sur le puits d'évacuation 10 fixé à la paroi inférieure du compartiment 3a.

Dans certains cas, il peut être avantageux de monter le puits d'évacuation dans le logement 45 qui contient le dispositif d'aspiration/injection 7, et de le porter au-dessous de l'embout 47, par pivotement ou glissement, à l'aide de tous moyens appropriés à cet effet, lorsque cet embout est en position intermédiaire, tandis que les opérations d'aspiration/injection s'effectuent en position basse : cette variante est intéressante parce qu'elle réduit les mouvements de la cassette 11 qui sont nécessaires, lorsque le puits est fixé sur le fond du compartiment 3a, toutes les fois que l'on veut évacuer le ou les produits aspirés pour aligner l'ouverture centrale du plateau 4 avec le dispositif d'aspiration/injection et le puits d'évacuation.

Lorsque l'embout 47, qui est réalisé en métal, en verre ou en matière plastique (transparente ou non), et qui est à la fois un réservoir et une aiguille, vient se placer au-dessus d'une logette 25, il est descendu par le dispositif 46 qui est en position intermédiaire (ou basse selon la variante précédente). L'extrémité de l'embout s'arrête à environ 2 mm du fond de la logette 25.

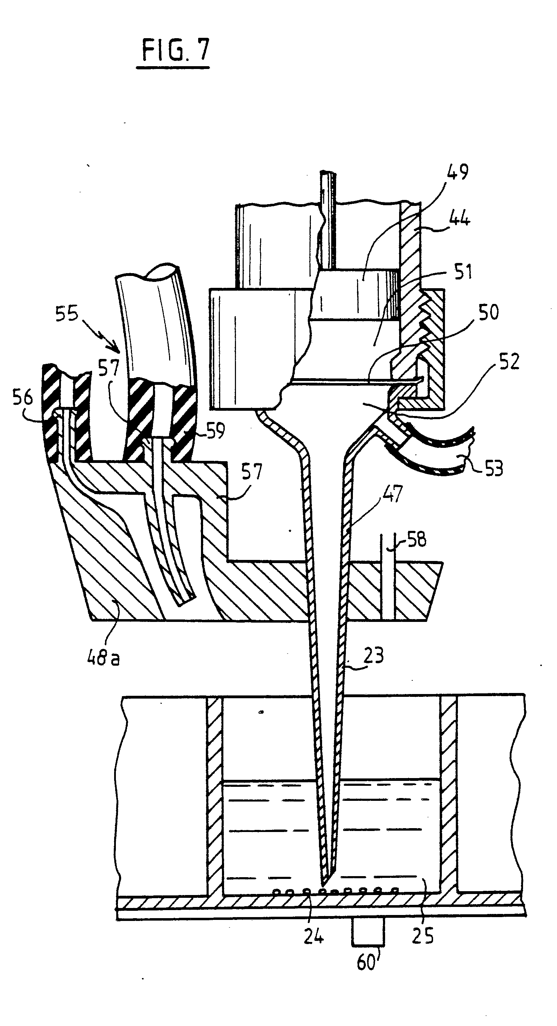

L'aspiration a lieu par retrait du piston 49 (Cf. la figure 7) qui aspire la membrane 50 par l'intermédiaire de l'espace 51 qui, suivant le type de manipulation, peut être rempli d'air ou d'un liquide inerte comme l'eau ou l'huile. La variation de l'espace 51 permet de régler l'aspiration et de la rendre plus ou moins énergique. La dépression ainsi créée par la membrane 50 se transmet à l'espace 52 et aspire le liquide contenu dans le micro-puits 25. Lorsque le puits d'évacuation 10 est ménagé dans la paroi inférieure du compartiment 3a, la cassette 11 est déplacée horizontalement pour aligner avec l'embout 47 et le puits 10 l'ouverture centrale du plateau 4. Le dispositif 46 est alors porté en position basse et la collerette 48 vient s'appliquer contre la surface supérieure du puits 10 pour créer l'étanchéité. Le piston 49 est alors repoussé pour chasser le liquide contenu dans 52. Par l'intermédiaire de la prise 53, de l'eau distillée stérile est injectée pour nettoyer l'intérieur de l'embout. Des perforations 54 placées à l'intérieur du puits 10 permettent d'injecter de l'eau pour nettoyer l'extérieur de l'embout 47. Ce rinçage peut être plus énergique et faire appel à de la vapeur d'eau, de l'alcool, etc...

Après l'évacuation et le rinçage, le dispositif 46 est porté en position haute pour laisser toute liberté de mouvement à la cassette 11 et au plateau 4.

On comprend aisément qu'il est possible de prélever, par ce dispositif, des quantités variables de liquide et de les replacer dans des micro-puits après avoir fait tourner le plateau 4. Pour éviter les pertes de gaz et maintenir les conditions stables dans le compartiment 3a, un clapet (non représenté) peut fermer la base du puits 10 et s'ouvrir par l'action même de l'embout 47, de longueur appropriée. La forme de l'extrémité de l'embout est biseautée. Le diamètre externe est d'environ 2 mm et le conduit central d'environ 0,05 mm. En élevant le plateau 4 en position haute à l'aide dudit bloc moteur 5, on amène au contact l'embout 47 et la culture cellulaire. Un mouvement alternatif très réduit du plateau permet de décoller les cellules et de les aspirer en vue de leur analyse et surtout de leur repiquage dans des micro-puits libres.

La figure 7 montre également comment une collerette 48a, de forme spéciale, enfilée sur l'embout 47 et collée supporte un dispositif d'injection 55 séparé dudit dispositif d'aspiration (qui peut en fait être également utilisé pour l'injection). Il comprend une arrivée d'air stérile et chaud 56 prélevée après le ventilateur du compartiment 3b. Une arrivée 57 permet l'injection de liquide nutritif. La canalisation d'air concentriquement à la base 57 évite le reflux de particules contaminantes. Une fibre optique 58 éclaire le micro-puits 25 en infra-rouge et une diode 60 mesure la lumière transmise, ce qui permet le contrôle du niveau. Un tuyau 59 isolant évite les condensations sur le tuyau froid qui arrive à la base 57.

Après aspiration du milieu nutritif et avant son évacuation dans le puits 10, les liquides neufs contenus dans le réservoir refroidi par l'enceinte réfrigérée 2 (Cf. la figure 1) s'écoulent directement lorsque des clapets étrangleurs 61 (cf. la figure 1) placés sous le réservoir, s'ouvrent. Le jet est dirigé contre l'embout 47, ce qui réalise à la fois un rinçage et évite l'impact direct du jet contre les cellules contenues au fond du micro-puits 25. L'écoulement du flacon est arrêté lorsque le signal arrivant à la diode réceptrice 56 indique une certaine valeur qui, en réalité, tient compte de la valeur initiale avant aspiration, de la valeur après aspiration, puis de l'évolution des mesures en cours de remplissage. La séquence de vidange et de nettoyage se déroule comme décrit précédemment.

En ce qui concerne les applications principales de l'automate selon l'invention, celles-ci concernent :

- le clonage : dans ce cas, le plateau 4 est disposé dans la cassette 11 après avoir ensemencé un seul micro-puits 25 ; à partir de l'identification des colonies, on repique et on garnit progressivement les autres micro-puits du plateau à l'aide dudit dispositif d'aspiration/injection ;

- l'analyse de l'action de substances toxiques, pharmacologiques ou autres : la rapidité du système selon l'invention permet de pratiquer des mesures à intervalles très rapprochés (du nombre, de la forme, de la cinétique cellulaire, etc...), et

- l'analyse de bactéries par réactions colorimétriques.

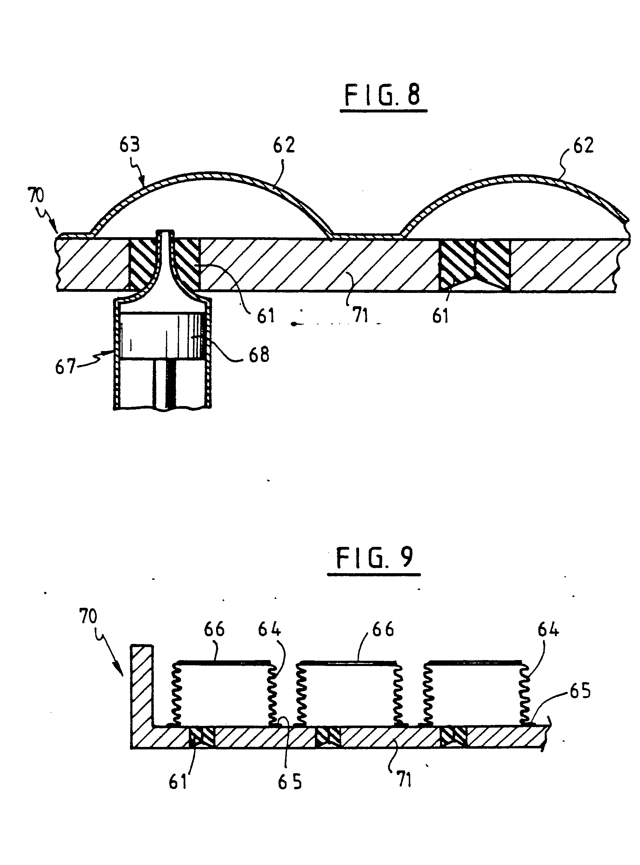

Les figures 8 et 9 se réfèrent à une variante 70 du plateau décrit précédemment qui convient à certaines applications parmi lesquelles on peut citer — et ce de manière non limitative — les expériences biologiques embarquées, à savoir en présence d'un environnement de micro-gravité ou d'absence totale de gravité (toutefois, certaines expériences sur Terre sont également susceptibles d'être effectuées en utilisant la variante de réalisation du plateau à micro-puits schématiquement illustrée aux figures 8 et 9).

Conformément à cette variante, le plateau 70 comporte des micro-puits fermés et à volume variable et l'accès du dispositif d'aspiration/injec- tion à l'intérieur de chaque micro-puits se fait par l'intermédiaire de pastilles 61, notamment en élastomère de silicone, qui sont pré-perforées ou perforables pour le passage d'une seringue 67 de type classique et notamment métallique dont la stérilisation peut être obtenue par chauffage, en particulier à l'aide d'une résistance électrique incorporée dans son piston 68 (ou dans sa paroi cylindrique) ou par chauffage IR ou autre moyen équivalent. Les pastilles 61 sont excentrées par rapport à l'axe de chaque micro-puits.

La figure 8 montre schématiquement un premier mode de réalisation des micro-puits à volume variable, suivant lequel les micro-puits sont constitués par des micro-bulles 62 sensiblement en forme de calotte sphérique, qui sont délimités par une membrane 63 transparente qui est collée sur la face supérieure du fond 71 du plateau 70 autour de chaque micro-bulle. Dans ce cas l'observation microscopique se fait du côté du fond 71.

La figure 9 montre également schématiquement une variante de réalisation des micro-puits à volume variable, qui sont définis par des micro-soufflets 64, eux aussi réalisés en matière élastique et transparente, qui sont collés sur la face supérieure du plateau 70 tout autour d'une collerette annulaire 65 ménagée au niveau du bord inférieur de chacun des micro-soufflets 64, ces derniers pouvant avantageusement être fermés par des opercules transparents 66 ayant des bonnes qualités optiques, ce qui permet dans ce cas d'effectuer l'observation microscopique du côté des micro-puits.

Dans certaines applications les micro-bulles et les micro-soufflets peuvent être avantageusement réalisés en une matière permettant les échanges gazeux, à savoir perméable aux gaz.

**Revendications**

1. Automate très compact pour l'analyse et le clonage de cultures cellulaires, ainsi que pour l'analyse bactériologique, à savoir intégrant les phases d'entretien, d'expérimentation de substances notamment pharmacologiques, d'analyse optique de la densité et de la forme, ainsi que de leurs associations, et de repiquage de cellules en vue de leur clonage, caractérisé en ce qu'il comprend, en combinaison :

- une enceinte (1) à parois isolantes, assurant l'homéothermie et certaines conditions d'ambiance, notamment en termes d'hygrométrie et de pourcentage de $CO_2$, à l'intérieur de cette enceinte, qui est divisée en deux compartiments (3a, 3b), dont le premier compartiment (3a) a un volume de l'ordre de grandeur du $dm^3$ et contient :

un plateau (4), de préférence circulaire, comprenant plusieurs micro-puits (25) disposés suivant une ou plusieurs rangées circulaires concentriques, le plateau (4) et les micro-puits (25) étant réalisés en une matière transparente ayant de bonnes qualités optiques,

au moins un dispositif spécial (6) d'observation microscopique, notamment du type à contraste de phase,

un dispositif (7) d'aspiration/injection de milieu cellulaire ou de cellules garantissant la non contamination,

un bloc moteur (5) assurant l'entraînement en rotation ainsi que les déplacements vertical et horizontal du plateau (4), avec positionnement très précis dans les trois directions de mouvement, tandis que le deuxième compartiment (3b) de l'enceinte (1), qui enveloppe le premier compartiment (3a), sauf au niveau de la face avant de l'enceinte, contient un dispositif de régulation de la température et de brassage de l'air chaud et, éventuellement, un dispositif de stérilisation du premier compartiment par flux d'air laminaire, filtré et en circuit fermé, connu en soi, lesdits premier et deuxième compartiments étant séparés par une paroi (9), éventuellement filtrante, pour permettre la circulation entre ces deux compartiments de l'éventuel flux laminaire, et

- un bloc réfrigérateur (2) disposé à l'extérieur de l'enceinte (1) ainsi équipée, contenant en particulier les réservoirs d'alimentation en milieux nutritifs et différents produits.

2. Automate selon la revendication 1, caractérisé en ce que ledit plateau (4) est pourvu d'une ouverture centrale d'accès dudit dispositif (7) d'aspiration/injection, qui est fixé à la paroi supérieure du premier compartiment (3a), dans un puits d'évacuation et de stérilisation (10) fixé à la paroi inférieure de ce compartiment (3a).

3. Automate selon l'une quelconque des revendications 1 et 2, caractérisé en ce que ledit plateau circulaire (4) est placé dans une cassette (11), notamment métallique et de forme sensiblement carrée qui, lorsqu'elle est équipée d'un couvercle, réalise une configuration proche de la boîte de Petri propre à réduire les risques de contamination et qui comporte un évidement circulaire central (12) de logement du plateau (4) qui, en position de repos, est tenu en place par des moyens de support constitués, de préférence, par quatre billes (21a, 21b) logées dans la paroi dudit évidement (12) et dépassant de cette dernière pour s'encliqueter dans une gorge annulaire (13) ménagée dans la paroi extérieure du plateau (4), tandis que l'entraînement en rotation du plateau (4) est réalisé à l'aide d'un galet moteur (14), faisant saillie dudit bloc moteur (5) par engagement, sous l'action de moyens appropriés, et par frottement dans la gorge (13) du plateau (4), à cet effet, une fenêtre (15) d'accès du galet (14) à cette gorge (13) est ménagée à la partie médiane du côté de la cassette (11) adjacent au bloc moteur (5).

4. Automate selon la revendication 3, caractérisé en ce que deux desdites billes (21a) sont fixes, tandis que les deux autres billes (21b) sont sur support élastique, notamment à ressort, les quatre billes (21a, 21b) étant disposées aux sommets d'un carré inscrit dans la circonférence délimitant ledit évidement circulaire (12), de manière à ce qu'un côté de ce carré, parallèle et contigu à ladite fenêtre (15) d'accès du galet moteur (14), est défini par les deux billes fixes, (21a), tandis que le côté opposé du carré est défini par lesdites deux billes (21b) sur support élastique, en sorte que, lors de l'engagement du galet (14) dans ladite gorge (13), ces deux dernières billes (21b) seulement participent au guidage en rotation du plateau (4), les deux billes fixes (21a) venant se dégager de la gorge (13) de ce dernier.

5. Automate selon la revendication 3, caractérisé en ce que lesdits moyens permettant l'engagement du galet moteur (14) dans la gorge (13) du plateau (4) sont constitués par un électro-aimant réalisant l'accouplement souple entre les parois métalliques opposées du bloc moteur (5) et de la cassette (11).

6. Automate selon la revendication 3, caractérisé en ce que la cassette de support (11) est pourvue d'un couvercle transparent (16), de bonne qualité optique, s'adaptant à ladite ouverture centrale du plateau (4) et pourvue d'une fenêtre radiale (17) d'accès dudit dispositif (7) d'aspiration/injection aux micro-puits (25) du plateau (4).

7. Automate selon la revendication 3, caractérisé en ce que des billes (20), disposées sur les parois latérales et sur les parois inférieure et supérieure de la cassette (11) permettent le libre glissement de cette cassette de support (11).

8. Automate selon la revendication 3, caractérisé en ce que la cassette de support (11) est stérilisable, de même que le plateau (4) et le couvercle (16).

9. Automate selon l'une quelconque des revendications 1 à 8, caractérisé en ce que celui-ci coopère avec une pluralité de plateaux (4), chacun contenu dans une cassette (11), du type susdit, de manière à stocker les cassettes dans une étuve à part et à n'introduire dans ledit premier compartiment (3a) que la cassette (11) correspondant à un certain protocole d'expérience.

10. Automate selon la revendication 1, caractérisé en ce que ledit bloc moteur (5) comprend, en combinaison :

- un premier moteur pas-à-pas (22) d'entraînement en rotation dudit plateau (4), qui agit sur ledit galet moteur (14) par l'intermédiaire d'un système réducteur (22a),

- un deuxième moteur pas-à-pas (30) qui assure le déplacement horizontal du plateau (4) en agissant sur un système d'engrenage qui fait saillie du bloc moteur (5) et qui agit à son tour sur au moins une crémaillère fixée à la paroi inférieure du premier compartiment (3a), et

- un troisième moteur pas-à-pas (26) de réglage unilatéral de la hauteur du plateau (4), qui agit sur une vis micrométrique (27) disposée latéralement par rapport audit galet moteur (14) et pourvue à son extrémité inférieure d'une bille de roulement (29) glissant sur la paroi inférieure (35) dudit premier compartiment (3a), de manière à permettre les déplacements horizontaux commandés par ledit deuxième moteur pas-à-pas (30).

11. Automate selon la revendication 1, caractérisé en ce que ledit dispositif d'observation microscopique comporte deux couples objectif/condenseur (33-34, 39-40) pré-réglés qui sont associés entre eux et qui sont destinés à fonctionner alternativement, les premier et deuxième condenseurs (34, 40) et les premier et deuxième objectifs (33, 39) étant solidaires du plafond et du plancher, respectivement, de la paroi de séparation entre lesdits deux compartiments (3a, 3b) de l'enceinte (1), lequel premier objectif (33) est à faible grossissement, tandis que le deuxième objectif (39) assure un grossissement moyen ou fort en fonction des besoins, l'arrivée de lumière dans les deux condenseurs (34, 40) se faisant par un système à fibres optiques (38).

12. Automate selon la revendication 11, caractérisé en ce que chaque objectif (33, 39) coopère avec un miroir (41, 42) et un capteur d'images constitué par une caméra de télévision (CT) ou par une barrette CCD couplé ou non à un analyseur d'images automatique.

13. Automate selon la revendication 12, caractérisé en ce que le miroir intermédiaire (42), qui est interposé entre ledit capteur d'images (CT) et le miroir (41) le plus éloigné de ce dernier, est semi-transparent.

14. Automate selon la revendication 12, caractérisé en ce que ledit miroir intermédiaire est escamotable, notamment sous l'action d'un système à électro-aimant, lorsque le couple objectif/condenseur (36, 39) le plus éloigné du capteur d'images (CT) est activé.

15. Automate selon la revendication 1, caractérisé en ce que le dispositif (7) d'aspiration/injection est disposé dans un logement vertical (45) ménagé dans la paroi supérieure du premier compartiment (3a) et coopère avec un dispositif (46) de réglage, à trois positions, de la hauteur de l'embout d'aspiration/injection (47), notamment constitué par un moteur pas-à-pas ou par un vérin télescopique, permettant :

- en position haute, de faire déplacer librement la cassette de support (11) ainsi que le plateau (4),

- en position intermédiaire, d'aspirer du milieu cellulaire appauvri ou des cellules, ou d'injecter du milieu enrichi ou des cellules dans un puits du plateau (10), l'éventuel contact entre la pointe dudit embout (47) et le fond du puits (10), étant obtenu par l'action dudit troisième moteur pas-à-pas (26) de réglage de la hauteur du plateau (4), et

- en position basse, d'évacuer dans ledit puits (10) d'évacuation, fixé à la paroi inférieure du premier compartiment (3a), le milieu appauvri aspiré et de procéder aux rinçages et à la stérilisation.

16. Automate selon la revendication 15, caractérisé en ce que l'embout (47) d'aspiration/injection est pourvu d'une collerette (48) faisant saillie de sa paroi externe qui, dans ladite position basse du dispositif (7) d'aspiration/injection, s'applique par pression sur le bord supérieur dudit puits (10) d'évacuation pour assurer l'étanchéité vis-à-vis des opérations de rinçage et de stérilisation de la partie externe de l'embout (47).

17. Automate selon la revendication 15, caractérisé en ce que, lorsque le dispositif (46) de réglage de la hauteur de l'embout (47) d'aspiration/injection est en position intermédiaire, ce dernier s'applique, au moyen de ladite collerette (48), sur le puits d'évacuation qui est contenu dans le même logement du dispositif d'aspiration/injection, le puits d'évacuation étant porté par pivotement ou par glissement au-dessous de l'embout (47) ainsi positionné par tous moyens appropriés, tandis que les opérations d'aspiration/injection s'effectuent en position basse.

18. Automate selon l'une quelconque des revendications 15 et 16 ou 15 et 17, caractérisé en ce que le dispositif d'injection est supporté par la collerette (48a) d'étanchéité, solidaire de l'embout (47) dudit dispositif d'aspiration, et comprend une arrivée d'air stérile et chaud (56) qui crée une colonne d'air laminaire autour de l'arrivée (57) de milieu nutritif injecté par des clapets (61), et qui empêche le reflux de particules contaminantes, à savoir les risques de contamination rétrograde.

19. Automate selon la revendication 18, caractérisé en ce que le dispositif d'injection coopère avec un dispositif de contrôle de niveau du milieu liquide injecté dans chaque micro-puits comportant une fibre optique (58) portée par ladite collerette (48a) et, en dessous de chaque micro-puits (25), une diode (60).

20. Automate selon l'une quelconque des revendications 1 à 19, caractérisé en ce qu'il coopère avec un microordinateur de pilotage de tous les dispositifs et de toutes les opérations qu'ils doivent accomplir suivant le protocole d'expérience prédéterminé, et qui assure également le traitement éventuel des données.

21. Automate selon la revendication 1, caractérisé en ce que le plateau (70) comporte des micro-puits qui sont fermés et à volume variable, l'accès du dispositif d'aspiration/injection à l'intérieur de chaque micro-puits se faisant par l'intermédiaire d'une pastille (61), notamment en élastomère de silicone, pré-perforée ou perforable, chaque pastille (61) étant incorporée dans le fond (71) du plateau (70), qui est transparent et présente des bonnes qualités optiques, et étant disposée de façon excentrique par rapport à l'axe du micro-puits correspondant.

22. Automate selon la revendication 21, caractérisé en ce que les micro-puits à volume variable sont constitués par des micro-bulles (62) sensiblement en forme de calotte sphérique, qui sont délimitées par une membrane élastique transparente (63) laquelle est collée sur la face supérieure du plateau (70) autour de chaque micro-bulle (62), dans ce cas l'observation microscopique se faisant du côté du fond (71) du plateau (70).

23. Automate selon la revendication 21, caractérisé en ce que les micro-puits à volume variable sont constitués par des micro-soufflets (64) en matière élastique et transparente qui sont collés sur la face supérieure du fond du plateau tout autour d'une collerette annulaire (65) ménagée au niveau de leur bord inférieur.

24. Automate selon la revendication 23, caractérisé en ce que les micro-soufflets (64) sont fermés chacun par un opercule transparent (66) et ayant des bonnes qualités optiques qui est intégré dans la paroi du micro-soufflet (64), dans ce cas l'observation microscopique se faisant du côté des micro-puits.

25. Automate selon l'une quelconque des revendications 22 et 24, caractérisé en ce que les micro-bulles (62) ou les micro-soufflets (64) sont réalisés en une matière élastique perméable au gaz.

26. Automate selon la revendication 21, caractérisé en ce que le dispositif d'aspiration/injection est constitué par une seringue classique métallique (67) permettant la stérilisation par chauffage, notamment à l'aide d'une résistance électrique incorporée ou par chauffage IR.

**Claims**

1. Very compact automatic apparatus for the analysis and cloning of cell cultures, and also for bacteriological analysis, namely combining the steps of maintenance, testing of substances, in particular pharmacological substances, optical analysis of the density and shape of the cells and of their groupings, and subculture of cells for the purpose of their cloning, characterised in that it comprises, in combination :
- an enclosure (1) with insulating walls ensuring homeothermy and certain environmental conditions, in particular in terms of hygrometry and percentage of $CO_2$, inside this enclosure, which is divided into two compartments (3a, 3b), of which the first compartment (3a) has a volume of the order of size of one $dm^3$ and contains :

a plate (4), preferably circular, comprising a plurality of microwells (25) disposed in one or more concentric circular rows, the plate (4) and the microwells (25) being made of a transparent material having good optical properties,

at least one special microscopic observation device (6), in particular of the phase-contrast type,

a suction/injection device (7) for cell medium or cells ensuring non-contamination,

a drive unit (5) ensuring the rotational driving and the vertical and horizontal movements of the plate (4), with very accurate positioning in the three directions of movement, while the second compartment (3b) of the enclosure (1), which surrounds the first compartment (3a), except at the level of the front face of the enclosure, contains a device for regulating the temperature of, and mixing, the warm air and, possibly, a device for sterilizing the first compartment by filtered laminar air flow in closed circuit, known per se, the said first and second compartments being separated by a wall (9), possibly a filtering wall, to permit circulation of the possible laminar flow between these two compartments, and
- a refrigerating unit (2) disposed outside the enclosure (1) equipped in this way and containing in particular the reservoirs for supplying nutrient media and various products.

2. Automatic apparatus according to claim 1, characterised in that the said plate (4) is provided with a central opening for access to the said suction/injection device (7), which is fixed to the top wall of the first compartment (3a), in an evacuation and sterilization well (10) fixed to the bottom wall of this compartment (3a).

3. Automatic apparatus according to either of claims 1 and 2, characterised in that the said circular plate (4) is placed in a cassette (11), in particular of metal and of substantially square shape, which, when it is equipped with a cover, produces a configuration approximating to a Petri dish and adapted to reduce the risks of contamination, and which comprises a central circular opening (12) for accommodating the plate (4) which, in a position of rest, is held in place by means of support preferably constituted by four small balls (21a, 21b) housed in the wall of the said opening (12) and projecting from the wall to catch in an annular groove (13) provided in the outer wall of the plate (4), while the rotational driving of the plate (4) is achieved with the aid of a driving roller (14) projecting from the said drive unit (5) by engagement, under the action of suitable means, and by friction in the groove (13) of the plate (4), a window (15) for access of the roller (14) to this groove (13) being arranged in the median portion of that side of the cassette (11) which is adjacent the drive unit (5).

4. Automatic apparatus according to claim 3, characterised in that two of the said balls (21a) are fixed, while the other two balls (27b) are on a resilient support, in particular of spring type, the four balls (21a, 21b) being disposed at the vertices of a square inscribed in the circumference defining the said circular opening (12), in such manner that one side of this square, parallel to and adjacent the said window (15) for access of the driving roller (14), is defined by the two fixed balls (21a), while the opposite side of the square is defined by the said two balls (21b) on a resilient support, so that, on the engagement of the roller (14) in the said groove (13), only these latter two balls (21b) participate in the guiding of the plate (4) during rotation, the two fixed balls (21a) disengaging themselves from the groove (13) in the plate.

5. Automatic apparatus according to claim 3, characterised in that the said means permitting engagement of the driving roller (14) in the groove (13) of the plate (4) are constituted by an electromagnet effecting flexible coupling between the opposed metal walls of the drive unit (5) and of the cassette (11).

6. Automatic apparatus according to claim 3, characterised in that the supporting cassette (11) is provided with a transparent cover (16) of good optical quality fitting to the said central opening in the plate (4) and provided with a radial window (17) for access of the said suction/injection device (7) to the microwells (25) of the plate (4).

7. Automatic apparatus according to claim 3, characterised in that small balls (20) disposed on the side walls and on the top and bottom walls of the cassette (11) allow free sliding of this supporting cassette (11).

8. Automatic apparatus according to claim 3, characterised in that the supporting cassette (11) is sterilizable, as are the plate (4) and the cover (16).

9. Automatic apparatus according to any one of claims 1 to 8, characterised in that it cooperates with a plurality of plates (4), each contained in a cassette (11), of the aforesaid type, so as to be able to store the cassettes in a separate oven and introduce into the said first compartment (3a) only the cassette (11) corresponding to a certain test procedure.

10. Automatic apparatus according to claim 1, characterised in that the said drive unit (5) comprises, in combination :
- a first stepping motor (22) for rotational driving of the said plate (4), which acts on the said driving roller (14) through the medium of a reducing system (22a),
- a second stepping motor (30) which ensures horizontal movement of the plate (4) by acting on a gear system which projects from the drive unit (5) and acts in turn on at least one rack fixed to the bottom wall of the first compartment (3a), and
- a third stepping motor (26) for unilateral adjustment of the height of the plate (4), which acts on a micrometric screw (27) disposed laterally with respect to the said driving roller (14) and provided at its lower end with a bearing ball (29) sliding on the bottom wall (35) of the said first compartment (3a), so as to allow the horizontal movements controlled by the said second stepping motor (30).

11. Automatic apparatus according to claim 1, characterised in that the said microscopic observation device comprises two preadjusted/objective/condenser pair (33-34, 39-40) which are associated with each other and are intended to function alternately, the first and second condensers (34, 40) and the first and second objectives (33, 39) being fast with the top and the bottom, respectively, of the separating wall between the said two compartments (3a, 3b) of the enclosure (1), which first objective (33) has low magnification, while the second objective (39) ensures medium or high magnification according to needs, the admission of light into the two condensers (34, 40) taking place through an optical fibre system (38).

12. Automatic apparatus according to claim 11, characterised in that each objective (33, 39) cooperates with a mirror (41, 42) and an image pick-up constituted by a television camera (CT) or by a CCD strip coupled or not to an automatic image analyser.

13. Automatic apparatus according to claim 12, characterised in that the intermediate mirror (42), which is interposed between the said image pick-up (CT) and the mirror (41) further from the latter, is semi-transparent.

14. Automatic apparatus according to claim 12, characterised in that the said intermediate mirror is retractable, in particular under the action of an electromagnet system, when the objective/condenser pair (33, 34) further from the image pick-up (CT) is activated.

15. Automatic apparatus according to claim 1, characterised in that the suction/injection device (7) is disposed in a vertical housing (45) provided in the top wall of the first compartment (3a) and cooperates with a three-position adjusting device (46) for the height of the suction/injection mouthpiece (47), this device being constituted in particular by a stepping motor or by a telescopic jack allowing :
- when in the high position, the supporting cassette (11) and the plate (4) to be caused to move freely,
- when in the intermediate position, impoverished cell medium or cells to be sucked up, or enriched medium or cells to be injected into a well (25) in the plate, contingent contact between the tip of the said mouthpiece (47) and the bottom of the well (25) being obtained by the action of the said third stepping motor (26) for adjusting the height of the plate (4), and
- when in the low position, the impoverished medium sucked up to be removed into the said evacuation well (10) fixed to the bottom wall of the first compartment (3a) and the rinsing operations and sterilization to be instituted.

16. Automatic apparatus according to claim 15, characterised in that the suction/injection mouthpiece (47) is provided with a flange (48) projecting from its outer wall, which flange, in the said low position of the suction/injection device (7), is applied by pressure against the top edge of the said evacuation well (10) to ensure tightness with respect to the operations of rinsing and sterilization of the outer part of the mouthpiece (47).

17. Automatic apparatus according to claim 15, characterised in that when the adjusting device (46) for the height of the suction/injection mouthpiece (47) is in the intermediate position, the latter is applied by means of the said flange (48) on the evacuation well, which is contained in the same housing as the suction/injection device, the evacuation well being brought below the mouthpiece (47) positioned in this way by any suitable means by pivoting or sliding, while the suction/injection operations are effected in the low position.

18. Automatic apparatus according to either of claims 15 and 16 or 15 and 17, characterised in that the injection device is supported by the sealing flange (48a), fast with the mouthpiece (47) of the said suction device, and comprises an inlet (56) for sterile and warm air, which creates a laminar air column around the inlet (57) for nutrient medium injected through valves (61) and prevents reflux of contaminating particles, that is to say the risks of back contamination.

19. Automatic apparatus according to claim 18, characterised in that the injection device cooperates with a monitoring device for the level of the liquid medium injected into each microwell comprising an optical fibre (58) borne by the said flange (48a) and, below each microwell (25), a diode (60).

20. Automatic apparatus according to any one of claims 1 to 19, characterised in that it cooperates with a microcomputer for handling all the devices and all the operations which they are to perform in accordance with the predetermined test procedure, and which also ensures the eventual processing of the data.

21. Automatic apparatus according to claim 1, characterised in that the plate (70) comprises microwells which are closed and of variable volume, access of the suction/injection device to the interior of each microwell being obtained through the medium of a preperforated or perforatable plug (61), in particular of silicone elastomer, each plug (61) being incorporated in the bottom (71) of the plate (70), which is transparent and has good optical properties, and being disposed eccentrically with respect to the axis of the corresponding microwell.

22. Automatic apparatus according to claim 21, characterised in that the variable-volume microwells are constituted by microblisters (62) substantially in the form of spherical cups which are defined by a transparent elastic diaphragm (63) which is cemented to the upper face of the plate (70) around each microblister (62), microscopic observation being carried out in this case from the direction of the bottom (71) of the plate (70).

23. Automatic apparatus according to claim 21, characterised in that the variable-volume microwells are constituted by microbellows (64) of elastic and transparent material which are cemented to the upper face of the bottom of the plate all around an annular collar (65) arranged on a level with their bottom edge.

24. Automatic apparatus according to claim 23, characterised in that the microbellows (64) are each closed by a transparent cover (66) having good optical properties which is integrated in the wall of the microbellows (64), microscopic observation being carried out in this case from the direction of the microwells.

25. Automatic apparatus according to either of claims 22 and 24, characterised in that the microblisters (62) or the microbellows (64) are made of a gas-permeable elastic material.

26. Automatic apparatus according to claim 21, characterised in that the suction/injection device is constituted by a conventional metal syringe (67) permitting sterilization by heating, in particular with the aid of an incorporated electric resistor or by IR heating.

**Patentansprüche**

1. Sehr kompakte automatische Vorrichtung für die Analyse und das Klonen von Zellkulturen, wie auch für die bakteriologische Analyse, nämlich eine solche, welche die Phasen der Unterhaltung, des Experimentierens mit, insbesondere pharmakologischen, Substanzen, die optische Analyse der Dichte und der Form, sowie ihre Verbindungen, und die Versetzung von Zellen im Hinblick auf ihre Klonierung integriert, dadurch gekennzeichnet, daß sie in Kombination folgendes umfaßt :

- einen umschlossenen Raum (1) mit isolierenden Wänden, welche Homeothermie und gewisse Umgebungsbedingungen, insbesondere in Zuständen der Hygrometrie und des Prozentsatzes an $CO_2$, im Inneren dieses umschlossenen Raums sicherstellen, der in zwei Abteilungen (3a, 3b) unterteilt ist, von denen die erste Abteilung (3a) ein Volumen in der Größenordnung von $dm^3$ hat und folgendes enthält :

eine Platte (4), vorzugsweise kreisförmig, die mehrere Mikroschächte (25) enthält, welche gemäß einer oder mehrerer konzentrischer kreisförmiger Reihen angeordnet sind, wobei die Platte (4) und die Mikroschächte (25) aus einem transparenten Material hergestellt sind, das gute optische Eigenschaften hat,

wenigstens eine Spezialeinrichtung (6) für die mikroskopische Beobachtung, insbesondere vom Phasenkontrasttyp,

eine Einrichtung (7) zum Ansaugen/Injizieren von Zellmittel oder von Zellen, welche die Nichtkontamination garantiert,

einen Motorblock (5), welcher den Rotationsantrieb, wie auch die Vertikal- und Horizontalverlagerungen der Platte (4) mit sehr genauer Positionierung in den drei Bewegungsrichtungen sicherstellt,

während die zweite Abteilung (3b) des umschlossenen Raums (1), welche die erste Abteilung (3a), ausgenommen auf dem Niveau der Vorderseite des umschlossenen Raums, einschließt, eine Einrichtung zur Regelung der Temperatur und zur Durchwirbelung der Warmluft und, eventuell, eine Einrichtung zur Sterilisation der ersten Abteilung durch laminare, filtrierte und im geschlossenen Kreislauf erfolgende Strömung von Luft, wie an sich bekannt, enthält, wobei die erste und zweite Abteilung durch eine Wand (9) getrennt sind, die eventuell filtrierend ist, um die Zirkulation zwischen den beiden Abteilungen der eventuellen laminaren Strömung zu ermöglichen, und

- einen im Äußeren des so ausgerüsteten umschlossenen Raums (1) angeordneten Kühlblock (2), der insbesondere die Behälter für die Versorgung mit Nährböden und verschiedenen Produkten enthält.

2. Automatische Vorrichtung nach Anspruch 1, dadurch gekennzeichnet, daß die Platte (4) mit einer mittigen Zugangsöffnung der Einrichtung (7) zum Ansaugen/Injizieren, die an der oberen Wand der ersten Abteilung (3a) befestigt ist, in einen Schacht (10) für die Entleerung und die Sterilisation, der an der unteren Wand dieser Abteilung (3a) befestigt ist, versehen ist.

3. Automatische Vorrichtung nach irgendeinem der Ansprüche 1 und 2, dadurch gekennzeichnet, daß die kreisförmige Platte (4) in einer, insbesondere metallischen und in im wesentlichen viereckiger Form ausgebildeten, Kassette (11) angeordnet ist, welche, wenn sie mit einer Abdekkung ausgerüstet ist, eine Konfiguration realisiert, die nahe der Petrischale ist, geeignet, die Gefahren der Kontamination zu vermindern, und die eine mittige kreisförmige Aussparung (12) für die Unterbringung der Platte (4) beinhaltet, welche in Ruheposition mit Haltemitteln an Ort und Stelle gehalten wird, die vorzugsweise von vier Kugeln (21a, 21b) gebildet sind, die in der Wand der Aussparung (12) untergebracht sind und von letzterer vorstehen, um in eine ringförmige Nut (13) einzuschnappen, die in der äußeren Wand der Platte (4) angebracht ist, während der Rotationsantrieb der Platte (4) mit Hilfe einer Motorrolle (14), welche von dem Motorblock (5) vorsteht, durch Eingriff unter der Wirkung von geeigneten Mitteln und durch Reibung in der Nut (13) der Platte (4) realisiert ist, wobei zu diesem Zweck ein Fenster (15) für den Zugang der Rolle (14) zu dieser Nut (13) in dem mittleren Teil der Seite der Kassette (11) benachbart dem Motorblock (5) angeordnet ist.

4. Automatische Vorrichtung nach Anspruch 3, dadurch gekennzeichnet, daß zwei der Kugeln (21a) befestigt sind, während die beiden anderen Kugeln (21b) auf einem elastischen Träger, insbesondere mit Federkraft, sind, wobei die vier Kugeln (21a, 21b) an den Spitzen eines Quadrats angeordnet sind, das in den Umfang eingeschrieben ist, welcher die kreisförmige Aussparung (12) begrenzt, derart, daß die eine Seite dieses Quadrats, die parallel und benachbart zu dem Fenster (15) für den Zugang der Motorrolle (14) ist, von den beiden festen Kugeln (21a) begrenzt ist, während die gegenüberliegende Seite des Quadrats von den erwähnten beiden Kugeln (21b) auf elastischem Träger begrenzt ist, in der Weise, daß während des Eingriffs der Rolle (14) in der Nut (13) diese beiden letzteren Kugeln (21b) allein an der Rotations-führung der Platte (4) teilnehmen, während die beiden festen Kugeln (21a) außer Eingriff mit der Nut (13) der letzteren treten.

5. Automatische Vorrichtung nach Anspruch 3, dadurch gekennzeichnet, daß die erwähnten Mittel, welche den Eingriff der Motorrolle (14) in der Nut (13) der Platte (4) gestatten, von einem Elektromagneten gebildet sind, der die elastische Ankupplung zwischen den gegenüberliegenden metallischen Wänden des Motorblocks (5) und der Kassette (11) bewirkt.

6. Automatische Vorrichtung nach Anspruch 3, dadurch gekennzeichnet, daß die Trägerkassette (11) mit einer transparenten Abdeckung (16) von guten optischen Eigenschaften versehen ist, die sich an die mittige Öffnung der Platte (4) anpaßt und mit einem radialen Fenster (17) für den Zugang der Einrichtung (7) zum Ansaugen/Injizieren zu den Mikroschächten (25) der Platte (4) versehen ist.

7. Automatische Vorrichtung nach Anspruch 3, dadurch gekennzeichnet, daß Kugeln (20), die auf den seitlichen Wänden und auf den unteren und oberen Wänden der Kassette (11) angeordnet sind, das freie Gleiten dieser Trägerkassette (11) gestatten.

8. Automatische Vorrichtung nach Anspruch 3, dadurch gekennzeichnet, daß die Trägerkassette (11) sterilisierbar ist, ebenso wie die Platte (4) und die Abdeckung (16).

9. Automatische Vorrichtung nach irgendeinem der Ansprüche 1 bis 8, dadurch gekennzeichnet, daß sie mit einer Mehrzahl von Platten (4) zusammenwirkt, von denen jede in einer Kassette (11) der besagten Art enthalten ist, derart, daß die Kassetten in einem Trockenschrank einzeln gespeichert werden und nur die Kassette (11) in die erste Abteilung (3a) eingeführt wird, welche einem gewissen Versuchsprotokoll entspricht.

10. Automatische Vorrichtung nach Anspruch 1, dadurch gekennzeichnet, daß der Motorblock (5) in Kombination folgendes umfaßt:
- einen ersten Schrittmotor (22) zum Rotationsantrieb der Platte (4), welcher über ein Untersetzungssystem (22a) auf die Motorrolle (14) wirkt,
- einen zweiten Schrittmotor (30), der die Horizontalverlagerung der Platte (4) sicherstellt, indem er auf ein Getriebesystem wirkt, das von dem Motorblock (5) vorsteht und seinerseits auf wenigstens eine Zahnstange wirkt, die an der unteren Wand der ersten Abteilung (3a) befestigt ist, und
- einen dritten Schrittmotor (26) für die einseitige Regulierung der Höhe der Platte (4), welcher auf eine Mikrometerschraube (27) wirkt, die seitlich bezüglich der Motorrolle (14) angeordnet und an ihrem unteren Ende mit einer Rollkugel (29) versehen ist, welche auf der unteren Wand (35) der ersten Abteilung (3a) gleitet, und zwar derart, daß horizontale Verlagerungen ermöglicht werden, die von dem zweiten Schrittmotor (30) gesteuert sind.

11. Automatische Vorrichtung nach Anspruch 1, dadurch gekennzeichnet, daß die Einrichtung zur mikroskopischen Beobachtung zwei voreingestellte Objektiv-/Kondensorpaare (33-34, 39-40) umfaßt, die miteinander verbunden sind und die dazu bestimmt sind, alternativ zu funktionieren, wobei der erste und zweite Kondensor (34, 40) und das erste und zweite Objektiv (33, 39) fest mit der Decke bzw. dem Boden der Trennwand zwischen den beiden Abteilungen (3a, 3b) des umschlossenen Raums (1) verbunden sind, wobei das erste Objektiv (33) für schwache Vergrößerung ist, während das zweite Objektiv (39) eine mittlere oder starke Vergrößerung in Abhängigkeit von den Bedürfnissen sicherstellt, wobei die Zuführung von Licht in die beiden Kondensoren (34, 40) durch ein faseroptisches System (38) erfolgt.

12. Automatische Vorrichtung nach Anspruch 11, dadurch gekennzeichnet, daß jedes Objektiv (33, 39) mit einem Spiegel (41, 42) und einem Bildaufnehmer, der von einer Fernsehkamera (CT) oder von einer geladenen angekoppelten Einrichtung, die an einen automatischen Bildanalysator angekoppelt ist oder nicht, gebildet ist, zusammenwirkt.

13. Automatische Vorrichtung nach Anspruch 12, dadurch gekennzeichnet, daß der zwischenliegende Spiegel (42), der zwischen den Bildaufnehmer (CT) und dem von letzteren entferntesten Spiegel (41) zwischengefügt ist, halbdurchlässig ist.

14. Automatische Vorrichtung nach Anspruch 12, dadurch gekennzeichnet, daß der zwischenliegende Spiegel, insbesondere durch Betätigung mit einem Elektromagnetsystem, einziehbar ist, wenn das Objektiv-/Kondensorpaar (33, 34), das vom Bildaufnehmer (CT) am entferntesten ist, aktiviert ist.

15. Automatische Vorrichtung nach Anspruch 1, dadurch gekennzeichnet, daß die Einrichtung (7) zum Ansaugen/Injizieren in einer vertikalen Unterbringung (45) angeordnet ist, die in der oberen Wand der ersten Abteilung (3a) angebracht ist, und mit einer drei Positionen aufweisenden Regulierungseinrichtung (46) für die Höhe des Ansatzstücks (47) zum Ansaugen/Injizieren zusammenwirkt, welche insbesondere von einem Schrittmotor oder einer teleskopischen Hebevorrichtung gebildet ist und folgendes gestattet :

- in der oberen Position eine freie Verlagerung der Trägerkassette (11) ebenso wie der Platte (4) durchzuführen,

- in der mittleren Position verarmtes Zellmittel oder Zellen anzusaugen oder angereichertes Mittel oder Zellen in einen Schacht (25) der Platte zu injizieren, wobei der eventuelle Kontakt zwischen der Spitze des Ansatzstücks (47) und dem Boden des Schachts (25) durch die Aktion des dritten Schrittmotors (26) für die Regulierung der Höhe der Platte (4) erhalten wird, und

- in der unteren Position in den Schacht (10) für die Entleerung, der an der unteren Wand der ersten Abteilung (3a) befestigt ist, das angesaugte verarmte Mittel zu entleeren und die Auswaschungen und die Sterilisation vorzunehmen.

16. Automatische Vorrichtung nach Anspruch 15, dadurch gekennzeichnet, daß das Ansatzstück (47) zum Ansaugen/Injizieren mit einem von seiner äußeren Wand vorstehenden Kragen (48) versehen ist, der sich in der erwähnten unteren Position der Einrichtung (7) zum Ansaugen/Injizieren durch Druck auf den oberen Rand des erwähnten Schachts (10) für die Entleerung anlegt, um die Dichtheit gegenüber den Operationen des Auswaschens und der Sterilisation des äußeren Teils des Ansatzstücks (47) sicherzustellen.

17. Automatische Vorrichtung nach Anspruch 15, dadurch gekennzeichnet, daß dann, wenn die Einrichtung (46) zur Regulierung der Höhe des Ansatzstücks (47) für das Ansaugen/Injizieren in mittlerer Position ist, sich letzteres mittels des Kragens (48) auf den Schacht für die Entleerung auflegt, der in der gleichen Unterbringung wie die Einrichtung zum Ansaugen/Injizieren enthalten ist, wobei der Schacht für die Entleerung durch Schwenken oder durch Gleiten unterhalb des Ansatzstücks (47) geführt wird, sowie durch jedes geeignete Mittel positioniert wird, während die Vorgänge des Ansaugens/Injizierens in der unteren Position stattfinden.

18. Automatische Vorrichtung nach irgendeinem der Ansprüche 15 und 16 oder 15 und 17, dadurch gekennzeichnet, daß die Vorrichtung zum Injizieren durch den Kragen (48a) für die Dichtigkeit, der fest mit dem Ansatzstück (47) der Vorrichtung zum Ansaugen verbunden ist, getragen wird, und eine Zuführung (56) von steriler und warmer Luft umfaßt, die eine Säule laminarer Luft um die Zuführung (57) des Nährbodens erzeugt, welcher durch Sperrventile (61) injiziert wird, und welche den Rückfluß von kontaminierenden Teilchen verhindert, nämlich die Gefahren einer rückwärtigen Kontamination.

19. Automatische Vorrichtung nach Anspruch 18, dadurch gekennzeichnet, daß die Einrichtung zum Injizieren mit einer Einrichtung zur Kontrolle des Niveaus des in jeden Mikroschacht injizierten flüssigen Mittels zusammenwirkt, welche eine Faseroptik (58) beinhaltet, die von dem erwähnten Kragen (48a) getragen ist, und unter jedem Mikroschacht (25) eine Diode (60).

20. Automatische Vorrichtung nach irgendeinem der Ansprüche 1 bis 19, dadurch gekennzeichnet, daß sie mit einer Mikrodatenverarbeitungsanlage zum Steuern aller Einrichtungen und aller Operationen, die gemäß dem vorbestimmten Versuchsprotokoll durchgeführt werden sollen, und die auch die eventuelle Verarbeitung der Daten sicherstellt, zusammenarbeitet.

21. Automatische Vorrichtung nach Anspruch 1, dadurch gekennzeichnet, daß die Platte (70) Mikroschächte beinhaltet, die geschlossen und von variablem Volumen sind, wobei der Zugang der Einrichtung zum Ansaugen/Injizieren zum Inneren von jedem Mikroschacht mittels eines Plättchens (61), insbesondere aus Siliconelastomer, erfolgt, das vorperforiert oder perforierbar ist, wobei jedes Plättchen (61) in den Boden (71) der Platte (70) inkorporiert ist, welcher transparent ist und gute optische Eigenschaften hat, und das in exzentrischer Weise bezüglich der Achse des entsprechenden Mikroschachts angeordnet ist.

22. Automatische Vorrichtung nach Anspruch 21, dadurch gekennzeichnet, daß die Mikroschächte mit variablem Volumen von Mikroblasen (62) im wesentlichen in der Form einer Kugelkalotte gebildet sind, welche von einer transparenten elastischen Membran (63) begrenzt sind, die auf der oberen Seite der Platte (70) um jede Mikroblase (62) geklebt ist, wobei in diesem Fall die mikroskopische Beobachtung von der Seite des Bodens (71) der Platte (70) her erfolgt.

23. Automatische Vorrichtung nach Anspruch 21, dadurch gekennzeichnet, daß die Mikroschächte von variablem Volumen von Mikrobälgen (64) aus elastischem und transparentem Material gebildet sind, welche auf die obere Fläche des Bodens der Platte ganz um einen ringförmigen Kragen (65) herum geklebt sind, der auf dem Niveau ihres unteren Rands angeordnet ist.

24. Automatische Vorrichtung nach Anspruch 23, dadurch gekennzeichnet, daß die Mikrobälge (64) jeder durch einen transparenten und gute optische Eigenschaften besitzenden Deckel (66) geschlossen sind, der in die Wand des Mikrobalgs (64) integriert ist, wobei in diesem Fall die mikroskopische Beobachtung von der Seite der Mikroschächte her erfolgt.

25. Automatische Vorrichtung nach irgendeinem der Ansprüche 22 und 24, dadurch gekennzeichnet, daß die Mikroblasen (62) oder die Mikrobälge (64) aus einem elastischen Material hergestellt sind, das für Gas durchlässig ist.

26. Automatische Vorrichtung nach Anspruch 21, dadurch gekennzeichnet, daß die Einrichtung zum Ansaugen/Injizieren von einer klassischen metallischen Spritze (67) gebildet ist, welche die Sterilisation durch Erhitzung, insbesondere mit Hilfe eines eingebauten elektrischen Widerstands oder durch die IR-Erhitzung gestattet.

FIG.1

CO$_2$

0 171 319

FIG. 2

11 21b 12 21 b

21a 15 21a

FIG. 3

16

21b

11

4 13

FIG. 4

18 19

45

7

11

6 12 17 16

## FIG. 5b

## FIG. 5a

## FIG. 6

CT

# FIG. 7

## FIG. 8

## FIG. 9